# EUROPEAN PATENT SPECIFICATION

(11) **EP 2 168 550 B1**
(45) Date of publication and mention of the grant of the patent: **13.07.2016**
(21) Application number: 09011928.0
(22) Date of filing: 18.09.2009
(51) Int. Cl.: A61H 23/02, A61H 23/04, A61H 9/00, A61H 1/00, A47C 3/02, A61M 21/02, A47C 3/025, A61M 21/00

(54) **Relaxation apparatus**
Entspannungseinrichtung
Appareil de relaxation

(30) Priority: 25.09.2008 JP 2008246636
(43) Date of publication of application: 31.03.2010
(73) Proprietor: Panasonic Intellectual Property Management Co., Ltd., Osaka 540-6207 (JP)
(72) Inventor: Morikawa, Daisuke, Kadoma-shi, Osaka (JP); Takahashi, Tatsuya, Kadoma-shi, Osaka (JP); Nishimura, Yoshinari, Kadoma-shi, Osaka (JP); Michimori, Akihiro, Kadoma-shi, Osaka (JP); Kawamoto, Youji, Kadoma-shi, Osaka (JP)
(74) Representative: Samson & Partner Patentanwälte mbB

(56) References cited:
- WO-A1-2009/128362
- US-B1- 6 494 850

## Description

### Field of the Invention

The present invention relates to an apparatus for providing a user with a relaxing and refresh effect.

### Background of the Invention

A conventional relaxation apparatus, which provides a user seating on a chair-shaped body-supporting member with relaxing effect by rocking the user's body, is described in Japanese Patent Laid-open Publication No. 2000-42112 (hereinafter, referred to as the cited reference).

The relaxation apparatus in the cited reference has a rocking unit (a rocking mechanism in the cited reference) which rocks a body-supporting member back and forth and hence provides a user with a relaxing effect by rocking the body-supporting member with the rocking unit at a frequency of 1 Hz or less while gradually changing the frequency with fluctuations of 0.2 Hz or less.

In the above-mentioned relaxation apparatus, a relaxation effect is provided to a user by rocking the user's body, so that the user can be lead to sleeping with the physical tension relaxed, for example. However, there is no technology to give the user with an effective refresh effect, such as awakening, following the relaxation effect, and hence its development is in great demand.

US 6 494 850 B1 discloses a relaxation apparatus according to the preamble of claim 1.

### Summary of the Invention

In view of the above, the present invention provides a relaxation apparatus capable of giving a user a refresh effect after a relaxation effect to the user by rocking the user's body.

While the invention is defined in the independent claim 1, further aspects of the invention are set forth in the dependent claims, the drawings and the following description.

In accordance with an aspect of the present invention, there is provided a relaxation apparatus including a body-supporting unit with a seat portion on which a user can sit and a back portion is fixed at a rear part of the seat portion, the back portion being reclined about the rear part of the seat portion by a reclining mechanism, a rocking unit for rocking the body-supporting unit and a controller for controlling the rocking unit to rock the body-supporting unit, characterized in that: the controller controls the reclining mechanism to move the back porton in directions of raise and reclining alternatively to refresh the user in a relaxed state.

The controller may control the reclining mechanism to raise the back portion stepwise with time while moving the back portion in the directions of raise and reclining alternatively.

Further, the controller may control the reclining mechanism so that the amount of motion of the back portion gets greater in the direction of raise than in the direction of reclining.

In addition, the controller may control the reclining mechanism so that the speed of its motion gets higher in the direction of raise than in the direction of reclining.

Moreover, the controller may control the reclining mechanism so that the speed of its motion in the direction of raise increases gradually as the motions in the directions of raise and reclining alternate.

Furthermore, the controller may control the reclining mechanism so that the back portion is put to rest for a specific period of time when its motion changes the direction from reclining to raise and vice versa, and control the reclining mechanism so that the motion stops longer when the direction changes from raise to reclining than from reclining to raise.

For example, the body-supporting unit is rocked in forward and backward directions, and the controller may control the reclining mechanism so that the back portion moves in the direction of raise in accordance with the rocking motion of the body-supporting unit in the backward direction.

Further, the controller may control the rocking unit so that at least one of frequency and amplitude of the rocking motion increases in accordance with the motion of the back portion in the direction of raise.

The back portion may include an air bag that is inflated to press the body of the user and contracted by an air pump, and the controller may control the air pump to inflate the air bag when the back portion is moved in the direction of reclining by the reclining mechanism.

In accordance with the present invention, the relaxation apparatus is capable of giving a refresh effect to a user after giving the user a relaxation effect by rocking the user's body.

### Brief Description of the Drawings

Fig. 1 shows a schematic configuration of a relaxation apparatus in accordance with an embodiment of the present invention.
Fig. 2 illustrates a perspective view of a body-supporting member of the relaxation apparatus in Fig. 1.
Fig. 3 depicts a graph for explaining frequency and amplitude of a rocking motion of the body-supporting member.
Fig. 4 is a diagram for explaining a motion of a back portion of the body-supporting member.
Fig. 5 is a diagram for explaining a motion of the back portion in another example.
Fig. 6 is a diagram for explaining a motion of the back portion in still another example.
Fig. 7 is a diagram for explaining a speed of a motion of the back portion in still another example.
Fig. 8 is a diagram for explaining motions of the back portion and the body-supporting member in still another example.
Fig. 9 is a diagram for explaining frequencies of a motion of the back portion and a rocking motion in still another example.
Fig. 10 is a diagram for explaining a motion of the back portion and an inflation and contraction motion of an airbag in still another example.

### Detailed Description of the Embodiments

Hereinafter, embodiments of the present invention will be described in detail with reference to the accompanying drawings.

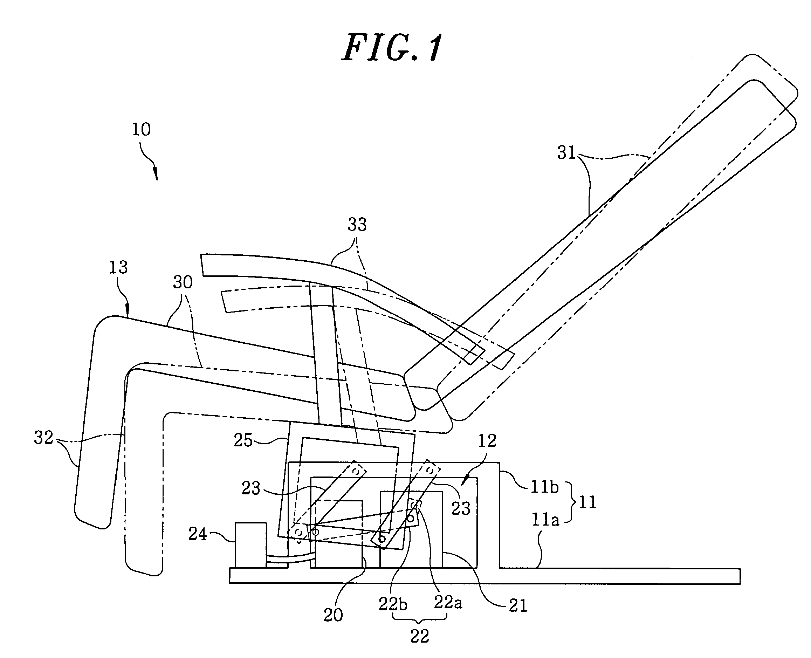

Fig. 1 shows a schematic configuration of a relaxation apparatus in accordance with an embodiment of the present invention. The relaxation apparatus 10 includes a base 11 having a bottom portion 11a placed on a floor (not shown), a rocking mechanism 12 as a rocking unit provided to the base 11 and a body-supporting member 13 as a body-supporting unit driven by the rocking mechanism 12.

The rocking mechanism 12 has a motor 20, a reduction gear 21, a crank mechanism 22 and a plurality of, e.g., two, link members 23. The motor 20 is provided on the bottom portion 11a of the base 11. The operation of the motor 20 is controlled by a controller 24 provided on the bottom portion 11a of the base 11. The reduction gear 21 is provided on the bottom portion 11a of the base 11. The reduction gear 21 is operationally coupled with the motor 20 and serves to reduce the power of the motor 20.

The crank mechanism 22 has two connecting rods 22a and 22b, and converts the rotating motion of the reduction gear 21 into a large circular motion. The base end of the connecting rod 22a is connected to and rotates with an output shaft of the reduction gear 21, while its leading end is ratatably connected to the base end of the connecting rod 22b. The leading end of the connecting rod 22b is connected to the lower portion of a rectangular frame 25 to which the body-supporting member 13 is fixed.

The base 11 also has a support frame 11b protruded upward from the bottom portion 11a. The base ends of the linkages 23 are rotatably connected to the upper portion of the support frame 11b at an interval, while their leading ends 22b are rotatably connected to the lower portion of the rectangular frame 25. The linkages 23 rotate around their base ends and, therefore, the body-supporting member 13, fixed to the rectangular frame 25, can be rocked back and forth by the power delivered from the crank mechanism 22, like a rocking chair. In the present embodiment of the invention, the movement path of the body-supporting member 13 from the rear to the front is referred to as a forward path and that from the front to the rear is referred to as a backward path.

The body-supporting member 13 of a chair shape includes a seat portion 30 which is fixed to an upper portion of the rectangular frame 25 so that they can move together, a back portion attached to the rear side of the seat portion 30 so that it can recline about the rear of the seat portion 30, an ottoman fixed at the front side of the seat portion 30 and two arm rests 33 fixed at both sides of the seat portion 30.

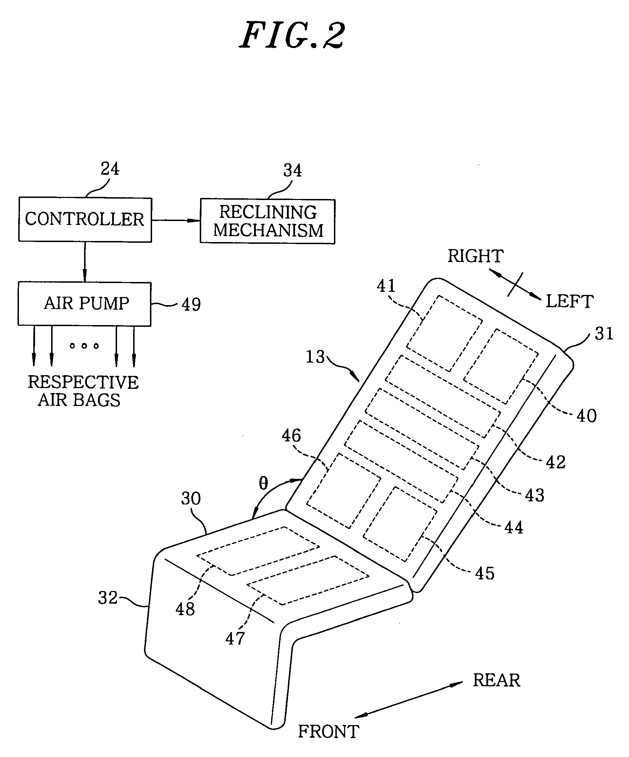

The back portion 31 can be reclined suitably by a reclining mechanism 34, which is controlled by the controller 24, as shown in Fig. 2.

In the upper part of the back portion 31, a pair of air bags 40 and 41 is provided corresponding respectively to the left and right shoulders of a user. In the middle of the back portion 31, three air bags 42, 43 and 44 are arranged vertically corresponding to the back (upper back, middle back and lower back) of a user. In the lower part of the back portion 31, two air bags 45 and 46 corresponding respectively to the left and right sides of the waist of a user. Two air bags 47 and 48 are provided at the left and right sides in the seat portion 30 corresponding to the hip and thigh of a user. The air bags 40 to 48 provided corresponding to respective body parts of a user, are installed to be activated by an air pump 49 provided under the seat portion 30 via connecting hoses (not shown). The operation of the air pump 49 is controlled by the controller 24 so that the air bags 40 to 48 can be inflated and contracted.

Hereinafter, the operation of the relaxation apparatus 10 configured as described above will be described with reference to Figs. 1 to 4.

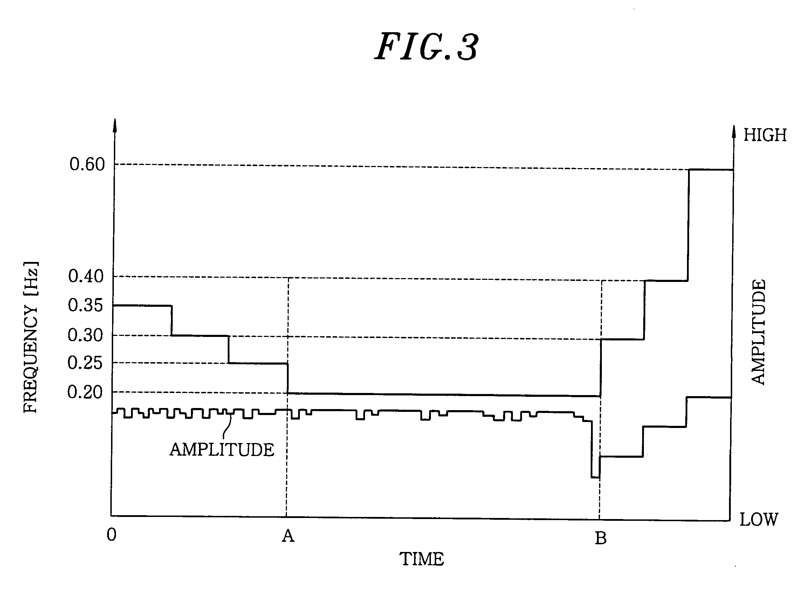

In the controller 24, several modes are provided including a single mode in which a relaxation rocking of the body-supporting member 13 is carried out by driving the rocking mechanism 12 and a combination mode in which the relaxation rocking is carried out in combination with operation of the respective air bags 40 to 48 and reclining of the back portion 31. Among them, in a relaxation-rocking mode having a refresh effect, a "relaxation rocking" is initiated to give a relaxation effect to a user for a predetermined period of time, after which the operation mode is switched from the "relaxation rocking" to "refresh rocking" until the operation mode ends. Fig. 3 shows the variations of amplitude and frequency with time of the motion of the body-supporting member 13 driven by the rocking mechanism 12 in the relaxation-rocking mode.

### (Relaxation rocking)

At first, the controller 24 controls the reclining mechanism 34 to recline the back portion 31 by a specified degree (for example, Δθ = 30°) from a normal state (for example, at θ = 120°) to a reclined state (for example, at θ = 150°), where θ denotes the angle the back portion 31 makes with the seat portion 30. During the reclining, the speed at which the back 31 is reclined is controlled to have a gradual decrease. In order to lead a user to be from an awakened state to a relaxed state, the controller 24 controls the motor 20 to decrease the frequency of rocking of the body-supporting member 13 gradually, for example, from its initial value 0.35 Hz to the final 0.2 Hz in a piecewise manner by a decrement of 0.05 Hz until a predetermined time denoted by A in Fig. 3. At the same time, the motor is controlled to introduce fluctuations to the amplitude of the rocking motion.

In addition, the controller 24 drives the air pump 49 until time A (Fig. 3) so as to inflate or contract appropriately the air bags 40 to 48 in the back portion 31 or the seat portion 30. A user's body gets soothed or extended so that he/she is provided with a massaging effect of relaxing muscles.

Until a predetermined time B after the time A has lapsed, the controller 24 controls the motor 20 to generate fluctuations in the amplitude of the body-supporting member 13 at the minimum value of the frequency, 0.2 Hz in the present embodiment, and to continue the rocking motion in order to let the user rest in sleep in the relaxation state. During the time interval between A and B, the controller 24 suspends the operation of the air pump 49 and the air bags 40 to 48 as well.

### (Refresh rocking)

After the lapse of time B, the controller 24 controls the motor 24 to increase the frequency of the rocking motion in a piecewise manner, for example, from 0.2 Hz to 0.3 Hz, 0.4 Hz and 0.6 Hz, in order to induce a user in the sleep (or relaxation) state to an awakened state. At this time, the controller 24 controls the frequency change timings, for example, such that the frequency changes every ten round trips the rocking motion of the body-supporting member 13 makes. Further, the controller 24 controls the motor 20 such that the amplitude increases stepwise in synchronization with the frequency change timings.

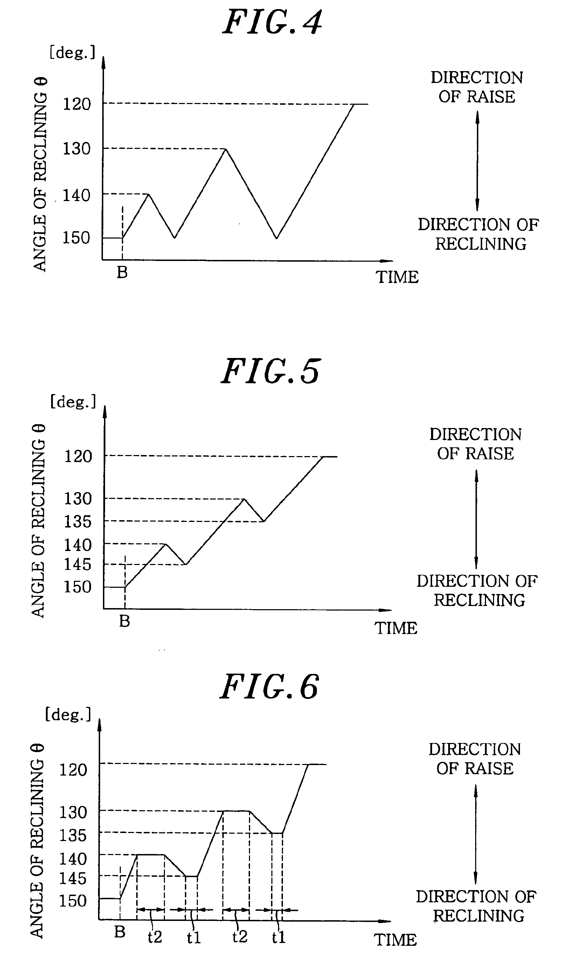

In parallel to the motion explained in the above, the controller 24 controls the reclining mechanism 34 such that the motion of the back portion 31 alternates its direction between raise (forward) and reclining (backward) as shown in Fig. 4. Specifically, the controller controls the reclining mechanism 34 so as to move the back portion 31 from its initial position at θ = 150° to θ = 140° in the direction of raise (forward) and then back to θ = 150° in the direction of reclining (backward), where θ is the angle of reclining defined as the angle the back portion 31 makes with the seat portion 30. Next, the controller controls the reclining mechanism 34 so as to move the back portion 31 from θ = 150° to θ = 130° in the direction of raise (forward) and then back to θ = 150° in the direction of reclining (backward). Finally, the controller controls the reclining mechanism 34 so as to move the back portion 31 from θ = 150° to θ = 120° in the direction of raise (forward), and in the case of a refresh rocking, the back portion 31 is configured to reach its final raised position at θ = 120°.

Thus, as the back portion 31 is reciprocated back and forth in the respective directions of reclining and raise, the upper body (in particular, the head) of a user moves and, in particular, the changing stimulation is given by a feeling of rocking due to the variation of a moving trajectory of the semicircular canal responsible for sense of equilibrium and the sensation of movement gets stimulated by body joints of the user, providing a refresh effect to him/her. In addition, because, as delineated in the above, the upper body of the user gets raised successively during the course of time by alternating the motion of the back portion 31 in the directions of raise (forward) and reclining (backward), an abrupt change in blood pressure of the user gets suppressed, so does the physical burden imposed upon him/her. By bringing the back portion 31 to its standing position at the end of the refresh rocking, the bust of the user is definitely in its standing position as well, providing him/her with a more effective refresh effect.

Besides, when the successive reclining motion of the back portion 31 driven by the reclining mechanism 34 ends, the controller 24 controls the air pump 49 so as to supply air to the air bags 42 to 44 and lift the back of the user, leading to a back stretching.

Next, the characteristic effects of the present embodiments will be described.
(1) The controller 24 controls the reclining mechanism 34 so as to successively reciprocate the back portion 31 in the directions of raise and reclining after the relaxation rocking, thereby refreshing a user in a relaxation state. In other words, as the back portion 31 is reciprocated back and forth in the respective directions of reclining and raise, the upper body (in particular, the head) of the user moves and, in particular, the changing stimulation is given by a feeling of rocking due to the variation of a moving trajectory of the semicircular canal responsible for sense of equilibrium and the sensation of movement gets stimulated by body joints of the user, providing a refresh effect to him/her.
(2) As the back portion 31 is reciprocated back and forth in the respective directions of reclining and raise, the reclining mechanism 34 is controlled by the controller 24 such that the back portion 31 gets raised successively during the course of time. In other words, by alternating the motion of the back portion 31 in the directions of raise and reclining successively but without having it arrive at the completely raised or reclined position, the bust of a user can be raised successively and hence an abrupt change in blood pressure of the user gets suppressed, so does the physical burden imposed upon him/her. Besides, by bringing the back portion 31 to its standing position at the end of the refresh rocking, the bust of the user is definitely in its standing position as well, providing him/her with a more effective refresh effect.

The embodiments of the present invention may be modified as described below.

In the embodiment described above, although during the refresh rocking motion, it is configured such that the amount of motion of the back portion 31 in the direction of raise is identical to that in the direction of reclining and it gets larger successively after each reciprocating cycle, the present invention is not limited thereto. For example, the amount of motion of the back portion may be larger in the direction of raise than in the direction of reclining as shown in Fig. 5, where the angle of reclining θ varies successively from its initial value θ = 150° to 140°, 145°, 130°, 135° and 120° during the course of the motion. Because the amount of motion is made larger in the direction of raise than in the direction of reclining, the angle of reclining approaches to the value of standing successively at the end of each reciprocating motion. For this reason, the bust of a user can be raised stepwise and hence an abrupt change in blood pressure of the user gets suppressed, so does the physical burden imposed upon him/her. Besides, since the back portion 31 is brought to its standing position during the course of time, the bust of a user gets slowly in its standing position, providing him/her with a more effective refresh effect.

Even though it is not mentioned explicitly in the embodiment described above, when the direction of reclining motion of the back portion 31 is changed by the controller 24 during the refresh rocking motion (after the given time B), the reclining mechanism 34 (see Fig. 2) may be configured to rest for a specific interval of time. In addition, as shown in Fig. 6, for example, the controller 24 (see Fig. 2) may control the reclining mechanism 34 such that the duration of standstill *t*₂ is longer when the direction of motion changes from raise to reclining than the duration of standstill *t*₁ when from reclining to raise. Because the back portion 31 can stand still longer at a relatively raised position in such a configuration, the bust of a user stays at a raised state closer to the vertical position, leading the user to a more suitably awakened state and hence providing him/her with a more effective refresh effect.

In addition, in the above configuration where the reclining mechanism 34 is controlled to have the duration of standstill *t*₂ longer than the duration of standstill *t*₁, the longer duration of standstill *t*₂ may increase successively with each reciprocating motion of the reclining mechanism, i.e., the back portion 31 may stay longer and longer at a raised position.

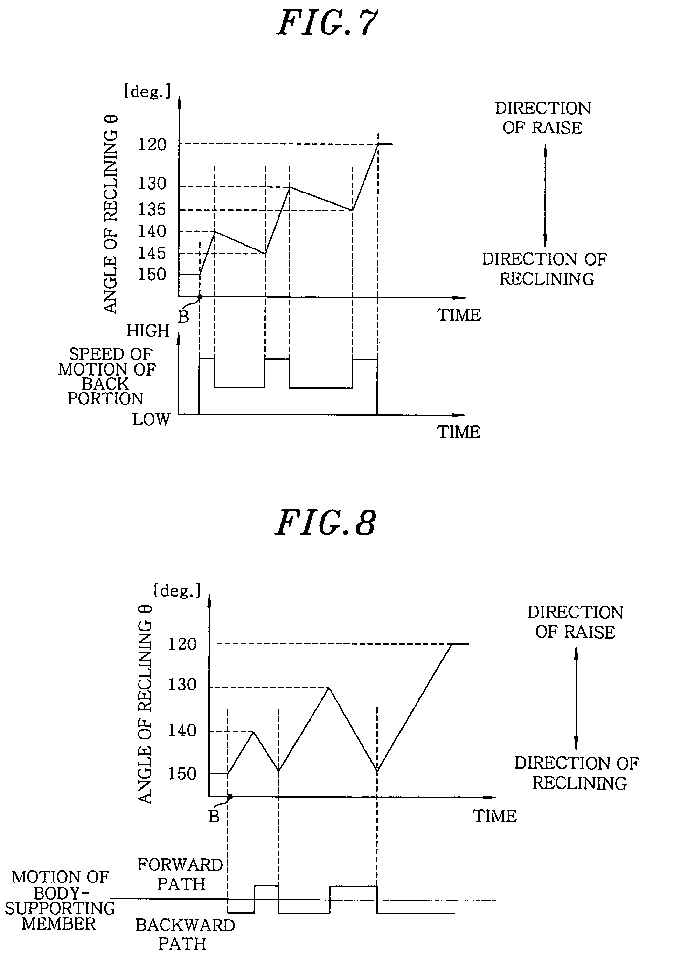

Even though it is not mentioned explicitly in the embodiment described above, it may be configured as shown in Fig. 7 that the speed of the motion of the back portion 31 is higher in the direction of raise than in the direction of reclining during the refresh rocking motion (after the given time B). In such a configuration, the motion of the back portion 31 is more noticeable in the direction of raise than in the direction of reclining and a user is more likely to feel the back portion being raised, providing him/her with a more effective refresh effect.

In addition, the reclining mechanism may be controlled by the controller 24 so as to increase the speed of motion of the back portion 31 in the direction of raise successively with each reciprocating motion of the reclining mechanism. In other words, during the course of time, the speed of motion of the back portion 31 in the direction of raise increases and a user is more likely to have a feeling of being raised. For this reason, a user in a relaxation state, being led to an awakened state in an abrupt manner, is led gradually to an awakened state, providing him/her with a more effective refresh effect.

Although it is not mentioned explicitly in the embodiment described above, it may be configured that, during the refresh rocking motion (after the given time B), the back portion 31 moves in the direction of raise while the body-supporting member 13 moves in the backward direction (along the backward path). In particular, the forward motion of the back portion 31 in the direction of raise may be made to take place in synchronization with the backward motion of the body-supporting member 13, as shown in Fig. 8. With the synchronization of the forward motion of the back portion 31 in the direction of raise with the backward rocking motion of the body-supporting member 13, a user more likely to have a feeling of being raised, providing him/her with a more effective refresh effect.

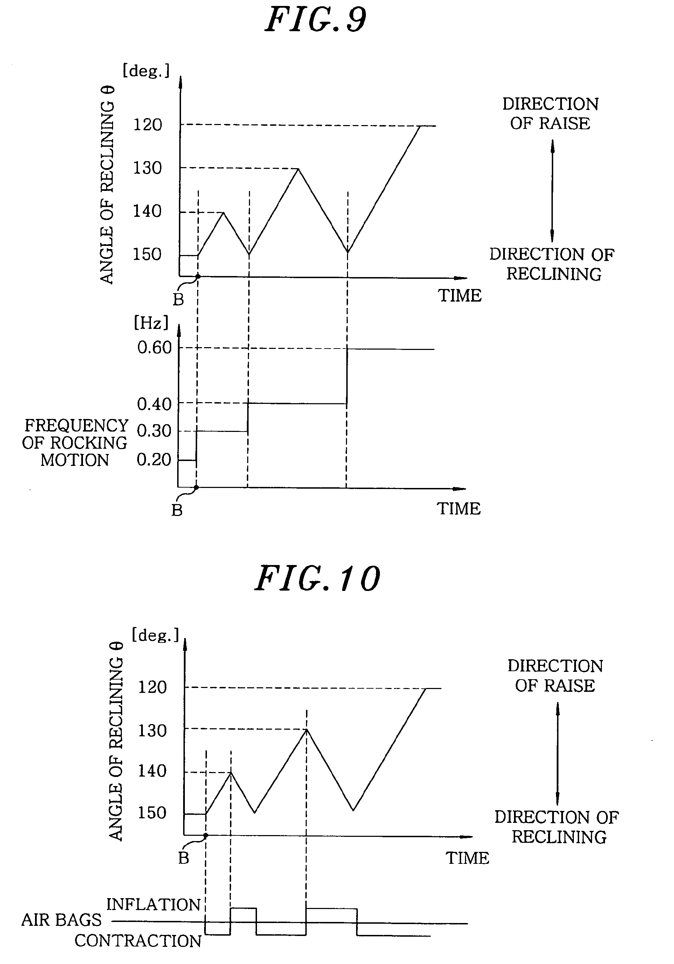

Although it is not mentioned explicitly in the embodiment described above, it may be configured that, during the refresh rocking motion (after the given time B), the back portion 31 is moved in the direction of raise in accordance with an increase in frequency of the rocking motion, as shown in Fig. 10, for example. Besides, the back portion 31 may be moved in the direction of raise in accordance with an increase in amplitude of the rocking motion. Because, in such a configuration, a change in at least one of the frequency and amplitude of the rocking motion takes place simultaneously at the inception of the motion in the direction of raise, a user is more likely to feel the change in motion of the body-supporting member 13 and the motion of the back portion 31 in the direction of raise, providing him/her with a more effective refresh effect.

Even though it is not mentioned explicitly in the embodiment described above, it may be configured that, during the refresh rocking motion (after the given time B), the back portion 31 moves in the direction of reclining in synchronization with inflation of the air bags 40 to 46, as shown in Fig. 10, for example. By such a configuration in which the air bags 40 to 46 are inflated in synchronization with the backward motion of the back portion 31 in the direction of reclining, a user is provided with a lessened feeling of motion in the direction of reclining and with an appropriate stimulation due to the weight of his/her bust that is applied upon the air bags 40 to 46, providing him/her with a more effective refresh effect. Besides, the same effect may be obtained by synchronizing inflation of at least one of the air bags 40 to 46 with the motion of the back portion 31 in the direction of reclining.

Although it is not mentioned explicitly in the embodiment described above, it may be configured that, during the refresh rocking motion (after the given time B), the back portion 31 moves in the direction of raise in synchronization with inflation of the air bags 40 to 46, for example. By such a configuration, the inflating motion of the air bags 40 to 46 is added to the motion of the back portion 31 in the same direction of raise to increase the amount of raise of the bust of a user, providing him/her with a more effective refresh effect.

In the embodiment described above, even though the back portion 31 reciprocates in the directions of reclining and raise at all times during the refresh rocking motion (after the given time B), it may be configured otherwise. For example, the back portion may move multiple times in the direction of raise before it moves once in the direction of reclining or vice versa.

In the embodiment described above, although it is configured that the body-supporting member 13 is rocked back and forth, the present invention is not limited thereto. For example, the body-supporting member 13 may be rocked sideways.

In the embodiment described above, while the body-supporting member 13 (the seat portion 30 and the back portion 31) includes the air bags 40 to 48, it may have no air bag.

In the embodiment described above, although it is configured that the controller 24 makes changes in control on the respective parts at the given times A and B, the present invention is not limited thereto. For example, there may be provided a switch capable of changing the control of the respective parts by the controller 24 so that a user can selectively control the respective parts by using the switch as needed.

In the embodiment described above, although it is not mentioned explicitly, there may be provided a mechanism for generating music or vibration corresponding to the relaxation rocking or refresh rocking.

## Claims

1. A relaxation apparatus (10) comprising a body-supporting unit (13) with a seat portion (30) on which a user can sit and a back portion (31) is fixed at a rear part of the seat portion (30), the relaxation apparatus further comprising a reclining mechanism and a rocking unit, the back portion (31) being reclinable about the rear part of the seat portion (30) by the reclining mechanism (34), the rocking unit (12) including a motor (20) for rocking the body-supporting unit (13) and a controller (24) for controlling the rocking unit (12) to rock the body-supporting unit (13), **characterized in that**:
the controller (24) provides an operation mode in which a relaxation rocking of the body-supporting unit (13) to give a relaxation effect to the user is carried out for a predetermined period of time and then a refresh rocking of the body-supporting unit (13) to give a refresh effect to the user is carried out until the operation mode ends;
the controller (24) is adapted to control, during the relaxation rocking, the reclining mechanism (34) to recline the back portion (31) by a specific degree from a standing position to a reclined state, a speed at which the back portion (31) is reclined to have a gradual decrease, the motor (20) to decrease a frequency of rocking of the body-supporting unit (13) gradually, and the motor (20) to introduce fluctuations to an amplitude of the rocking of the body-supporting unit (13); and
the controller (24) is adapted to control, during the refresh rocking, the reclining mechanism (34) to move the back portion (31) in the reclined state in directions of raise and reclining alternately until the back portion (31) reaches the standing position.

2. The relaxation apparatus (10) of claim 1, wherein the controller (24) is adapted to control, during the refresh rocking, the reclining mechanism (34) to raise the back portion (31) stepwise with time while moving the back portion (31) in the directions of raise and reclining alternatively.

3. The relaxation apparatus (10) of claim 1 or 2, wherein the controller (24) is adapted to control, during the refresh rocking, the reclining mechanism (34) so that the amount of motion of the back portion (31) gets greater in the direction of raise than in the direction of reclining.

4. The relaxation apparatus (10) of any one of claims 1 to 3, wherein the controller (24) is adapted to control, during the refresh rocking, the reclining mechanism (34) so that the speed of its motion gets higher in the direction of raise than in the direction of reclining.

5. The relaxation apparatus (10) of any one of claims 1 to 4, wherein the controller (24) is adapted to control, during the refresh rocking, the reclining mechanism (34) so that the speed of its motion in the direction of raise increases gradually as the motions in the directions of raise and reclining alternate.

6. The relaxation apparatus (10) of any one of claims 1 to 5, wherein during the refresh rocking, the controller (24) is adapted to control the reclining mechanism (34) so that the back portion (31) is put to rest for a specific period of time when its motion changes the direction from reclining to raise and vice versa, and controls the reclining mechanism (34) so that the motion stops longer when the direction changes from raise to reclining than from reclining to raise.

7. The relaxation apparatus (10) of any one of claims 1 to 6, wherein the body-supporting unit (13) is rocked in forward and backward directions, and
wherein the controller (24) is adapted to control, during the refresh rocking, the reclining mechanism (34) so that the back portion (31) moves in the direction of raise in accordance with the rocking motion of the body-supporting unit (13) in the backward direction.

8. The relaxation apparatus (10) of any one of claims 1 to 7, wherein the controller (24) is adapted to control, during the refresh rocking, the rocking unit (12) so that at least one of frequency and amplitude of the rocking motion increases in accordance with the motion of the back portion (31) in the direction of raise.

9. The relaxation apparatus (10) of any one of claims 1 to 6, wherein the back portion (31) includes at least one air bag (40, 41, 42, 43, 44, 45, 46) that is inflated to press the body of the user and contracted by an air pump (49), and
wherein the controller (24) is adapted to control, during the refresh rocking, the air pump (49) to inflate the air bag (40, 41, 42, 43, 44, 45, 46) when the back portion (31) is moved in the direction of reclining by the reclining mechanism (34).

## Patentansprüche

1. Entspannungsvorrichtung (10) umfassend eine Körperlagerungseinheit (13) mit einem Sitzabschnitt (30), auf dem ein Nutzer sitzen kann, und einem Rückenabschnitt (31), der an ein Hinterteil des Sitzbereichs (30) fixiert ist, wobei die Entspannungsvorrichtung weiter umfasst einen Zurücklehnmechanismus und eine Schaukeleinheit, wobei der Rückenabschnitt (31) zurücklehnbar um das Hinterteil des Sitzbereichs (30) über den Zurücklehnmechanismus (34) vorgesehen ist, wobei die Schaukeleinheit (12) einen Motor (20) zum Schaukeln der Körperlagerungseinheit (13) und eine Steuervorrichtung (24) zum Steuern der Schaukeleinheit (12), um die Körperlagerungseinheit (13) zu schaukeln, umfasst, **dadurch gekennzeichnet, dass**:
die Steuervorrichtung (24) einen Betriebsmodus vorsieht, in dem ein Entspannungsschaukeln der Körperlagerungseinheit (13) für eine vorher festgelegte Zeitperiode ausgeführt wird, um einen Entspannungseffekt an den Nutzer zu geben, und dann ein Auffrisch-Schaukeln der Körperlagerungseinheit (13), um einen Auffrischeffekt gegenüber dem Nutzer auszuführen, bis der Betriebsmodus endet, ausgeführt wird;
die Steuervorrichtung (24) ausgelegt ist, um während des Entspannungsschaukelns, den Zurücklehnmechanismus (34) zu steuern, so dass der Rückenabschnitt (31) um einen spezifischen Grad von einer aufrechten Position zu einem zurückgeneigten Zustand zurückzulehnen, um eine Geschwindigkeit zu steuern, mit der der Rückenabschnitt (31) zurückgelehnt wird, um eine graduell Verringerung aufzuweisen, um den Motor (20) zu steuern, um eine Schaukelfrequenz der Körperlagerungseinheit (13) graduell zu verringern, und um den Motor (20) zu steuern, um Fluktuationen zu einer Amplitude des Schaukelns der Körperlagerungseinheit (13) einzuführen; und
die Steuervorrichtung (24) ausgelegt ist, um den Zurücklehnmechanismus (34) während des Auffrischschaukelns derart zu steuern, um den Rückenabschnitt (31) in dem zurückgelehnten Zustand in Richtung eines Anhebens und Zurückneigens abwechselnd zu bewegen, bis der Rückenabschnitt (31) die aufrechte Position erreicht.

2. Entspannungsvorrichtung (10) gemäß Anspruch 1, bei der die Steuervorrichtung (24) ausgelegt ist, um während des Auffrisch-Schaukelns den Zurücklehnmechanismus (34) derart zu steuern, um den Rückenabschnitt (31) schrittweise mit der Zeit zu erheben, während der Rückenabschnitt (31) in die Richtungen eines Erhebens und eines Zurücklehnens abwechselnd bewegt wird.

3. Entspannungsvorrichtung (10) gemäß Anspruch 1 oder 2, bei der die Steuervorrichtung (24) ausgelegt ist, um während des Auffrisch-Schaukelns den Zurücklehnmechanismus (34) derart zu steuern, so dass die Bewegungsmenge des Rückenabschnitts (31) in der Richtung eines Anhebens größer wird als in die Richtung eines Zurückneigens.

4. Entspannungsvorrichtung (10) gemäß einem der Ansprüche 1 bis 3, bei der die Steuervorrichtung ausgelegt ist, um während des Auffrisch-Schaukelns den Zurücklehnmechanismus (34) derart zu steuern, so dass die Geschwindigkeit seiner Bewegung in die Richtung eines Anhebens höher wird als in die Richtung eines Zurückneigens.

5. Entspannungsvorrichtung (10) gemäß einem der Ansprüche 1 bis 4, bei der die Steuervorrichtung (24) ausgelegt ist, um während des Auffrisch-Schaukelns den Zurücklehnmechanismus (34) derart zu steuern, so dass die Geschwindigkeit seiner Bewegung in die Richtung eines Anhebens sich graduell erhöht, wenn die Bewegungen in die Richtung eines Anhebens und eines Zurückneigens sich abwechseln.

6. Entspannungsvorrichtung (10) gemäß einem der Ansprüche 1 bis 5, bei der die Steuervorrichtung (24) während des Auffrisch-Schaukelns ausgelegt ist, um den Zurücklehnmechanismus (34) derart zu steuern, so dass der Rückenabschnitt (31) für eine spezifische Zeitperiode eine Pause macht, wenn sich seine Bewegung die Richtung von einem Zurücklehnen zum Anheben und umgekehrt ändert, und den Zurücklehnmechanismus (34) derart steuert, so dass die Bewegung länger stoppt, wenn die Richtung sich von einem Anheben zu einem Zurücklehnen ändert, als von einem Zurücklehnen zu einem Anheben.

7. Entspannungsvorrichtung (10) gemäß einem der Ansprüche 1 bis 6, bei der die Körperlagerungseinheit (13) in Richtungen nach vorne und nach hinten geschaukelt wird, und
wobei die Steuervorrichtung (24) ausgelegt ist, um während des Auffrisch-Schaukelns den Zurücklehnmechanismus (34) derart zu steuern, so dass sich der Rückenabschnitt (31) in die Richtung eines Anhebens in Übereinstimmung mit der Schaukelbewegung der Körperlagerungseinheit (13) in der rückwärts gerichteten Richtung bewegt.

8. Entspannungsvorrichtung (10) gemäß einem der Ansprüche 1 bis 7, bei der die Steuervorrichtung (24) ausgelegt ist, um während des Auffrisch-Schaukelns die Schaukeleinheit (12) derart zu steuern, so dass wenigstens eine von einer Frequenz und einer Amplitude der Schaukelbewegung sich in Übereinstimmung mit der Bewegung des Rückenabschnitts (31) in der Richtung eines Anhebens erhöht.

9. Entspannungsvorrichtung (10) gemäß einem der Ansprüche 1 bis 6, bei der der Rückenabschnitt (31) wenigstens ein Luftkissen (40, 41, 42, 43, 44, 45, 46) umfasst, das aufgeblasen ist, um den Körper des Nutzers zu beaufschlagen und über eine Luftpumpe (49) zusammengezogen wird, und
wobei die Steuervorrichtung (24) ausgelegt ist, um während des Auffrisch-Schaukelns die Luftpumpe (49) derart zu steuern, um das Luftkissen (40, 41, 42, 43, 44, 45, 46) aufzublasen, wenn der Rückenabschnitt (31) über den Zurücklehnmechanismus (34) in die Richtung eines Zurücklehnens bewegt wird.

## Revendications

1. Appareil de relaxation (10) comprenant une unité de support de corps (13) avec une partie de siège (30) sur laquelle un utilisateur peut s'asseoir et une partie de dossier (31) est fixée au niveau d'une partie arrière de la partie de siège (30), l'appareil de relaxation comprenant en outre un mécanisme d'inclinaison et une unité de balancement, la partie de dossier (31) étant inclinable autour de la partie arrière de la partie de siège (30) grâce au mécanisme d'inclinaison (34), l'unité de balancement (12) comprenant un moteur (20) pour balancer l'unité de support de corps (13) et un organe de commande (24) pour commander l'unité de balancement (12) afin de balancer l'unité de support de corps (13), **caractérisé en ce que** :
l'organe de commande (24) fournit un mode de commande dans lequel un balancement de relaxation de l'unité de support de corps (13) pour donner un effet de relaxation à l'utilisateur, est réalisé pendant une période de temps prédéterminée et ensuite un balancement de détente de l'unité de support de corps (13) pour donner un effet de détente à l'utilisateur est réalisé jusqu'à ce que le mode de commande se termine ;
l'organe de commande (24) est adapté pour commander, pendant le balancement de relaxation, le mécanisme d'inclinaison (34) pour incliner la partie de dossier (31) avec un degré spécifique d'une position droite à un état incliné, une vitesse à laquelle la partie de dossier (31) est inclinée pour avoir une diminution progressive, le moteur (20) pour diminuer progressivement une fréquence de balancement de l'unité de support de corps (13), et le moteur (20) pour introduire des fluctuations à une amplitude du balancement de l'unité de support de corps (13) ; et
l'organe de commande (24) est adapté pour commander, pendant le balancement de détente, le mécanisme d'inclinaison (34) afin de faire passer la partie de dossier (31) à l'état incliné dans les directions de montée et d'inclinaison de manière alternée, jusqu'à ce que la partie de dossier (31) atteigne la position droite.

2. Appareil de relaxation (10) selon la revendication 1, dans lequel l'organe de commande (24) est adapté pour commander, pendant le balancement de détente, le mécanisme d'inclinaison (34) pour faire monter la partie de dossier (31) pas à pas avec le temps tout en déplaçant la partie de dossier (31) dans les directions de montée et d'inclinaison de manière alternée.

3. Appareil de relaxation (10) selon la revendication 1 ou 2, dans lequel l'organe de commande (24) est adapté pour commander, pendant le balancement de détente, le mécanisme d'inclinaison (34) de sorte que la quantité de mouvement de la partie de dossier (31) devient plus importante dans la direction de montée que dans la direction d'inclinaison.

4. Appareil de relaxation (10) selon l'une quelconque des revendications 1 à 3, dans lequel l'organe de commande (24) est adapté pour commander, pendant le balancement de détente, le mécanisme d'inclinaison (34) de sorte que la vitesse de son mouvement devient plus importante dans la direction de montée que dans la direction d'inclinaison.

5. Appareil de relaxation (10) selon l'une quelconque des revendications 1 à 4, dans lequel l'organe de commande (24) est adapté pour commander, pendant le balancement de détente, le mécanisme d'inclinaison (34) de sorte que la vitesse de son mouvement dans la direction de montée augmente progressivement au fur et à mesure que les mouvements dans les directions de montée et d'inclinaison alternent.

6. Appareil de relaxation (10) selon l'une quelconque des revendications 1 à 5, dans lequel, pendant le balancement de détente, l'organe de commande (24) est adapté pour commander le mécanisme d'inclinaison (34) de sorte que la partie de dossier (31) est placée au repos pendant une période spécifique de temps lorsque son mouvement passe de la direction d'inclinaison à la direction de montée et vice versa, et commande le mécanisme d'inclinaison (34) de sorte que le mouvement s'arrête plus longtemps lorsque la direction passe de la montée à l'inclinaison que de l'inclinaison à la montée.

7. Appareil de relaxation (10) selon l'une quelconque des revendications 1 à 6, dans lequel l'unité de support de corps (13) est balancée dans les directions avant et arrière, et
dans lequel l'organe de commande (24) est adapté pour commander, pendant le balancement de détente, le mécanisme d'inclinaison (34) de sorte que la partie de dossier (31) se déplace dans la direction de montée selon le mouvement de balancement de l'unité de support de corps (13) dans la direction arrière.

8. Appareil de relaxation (10) selon l'une quelconque des revendications 1 à 7, dans lequel l'organe de commande (24) est adapté pour commander, pendant le balancement de détente, l'unité de balancement (12) de sorte qu'au moins l'une parmi la fréquence et l'amplitude du mouvement de balancement augmente selon le mouvement de la partie de dossier (31) dans la direction de montée.

9. Appareil de relaxation (10) selon l'une quelconque des revendications 1 à 6, dans lequel la partie de dossier (31) comprend au moins un coussin d'air (40, 41, 42, 43, 44, 45, 46) qui est gonflé pour comprimer le corps de l'utilisateur et contracté par une pompe à air (49), et
dans lequel l'organe de commande (24) est adapté pour commander, pendant le balancement de détente, la pompe à air (49) qui gonfle le coussin d'air (40, 41, 42, 43, 44, 45, 46) lorsque la partie de dossier (31) est déplacée dans la direction d'inclinaison par le mécanisme d'inclinaison (34).
